# EUROPÄISCHE PATENTANMELDUNG

(11) **EP 2 314 344 A1**
(43) Veröffentlichungstag der Anmeldung: **27.04.2011**
(21) Anmeldenummer: 09013429.7
(22) Anmeldetag: 23.10.2009
(51) Int. Cl.: A61M 39/28, F16K 7/04, F16K 7/06

(54) **Elektrisch betätigbare Scherenklemme**

(71) Anmelder: LIFEBRIDGE Medizintechnik AG, 84539 Ampfing (DE)
(72) Erfinder: Brieske, Gerhard, 84539 Ampfing (DE)
(74) Vertreter: Manitz, Finsterwald & Partner GbR

(57) **Zusammenfassung**

Eine elektrisch betätigbare Klemme (12) zum Abklemmen von Schläuchen (10) umfasst einen elektrischen Antrieb (22), zwei Scherenhebel (16,16'), die gegeneinander um eine Schwenkachse (18) zwischen einer Öffnungsstellung und einer Klemmstellung verschwenkbar sind, und die jeweilige Klemmflächen (14,14') aufweisen, und eine Umsetzeinrichtung, mittels welcher eine Bewegung des elektrischen Antriebs in eine Bewegung der Scherenhebel umsetzbar ist.

## Beschreibung

Die Erfindung betrifft eine elektrisch betätigbare Klemme zum Abklemmen von Schläuchen.

In medizinischen Geräten, wie zum Beispiel Herz-Lungen-Maschinen, werden Flüssigkeiten, insbesondere Blut, mittels eines Schlauchsystems transportiert. Eine Herz-Lungen-Maschine dient unter anderem dazu, für einen bestimmten Zeitraum, beispielsweise während einer Herzoperation, die Funktion des Herzens und der Lunge eines Patienten zu übernehmen und den Blutkreislauf aufrechtzuerhalten. Dabei ist es von großer Wichtigkeit, dass keine Luftblasen transportiert werden, um ein lebensbedrohliches Eindringen von Luft in den Körperkreislauf des Patienten zu vermeiden. Daher kann in derartigen medizinischen Vorrichtungen an geeigneter Stelle ein Blasendetektor im Flüssigkeitskreislauf vorgesehen sein. Wird von diesem Detektor eine Luftblase im extrakorporalen Blutkreislauf festgestellt, so muss der Kreislauf sofort unterbrochen werden, damit eine Gefährdung des Patienten ausgeschlossen ist. Dazu dienen Klemmvorrichtungen, die an einem Schlauch des Flüssigkeitstransportsystems angreifen und diesen im Bedarfsfall abdrücken.

Das Abdrücken des Schlauches durch die Klemmvorrichtung muss dabei äußerst schnell vonstatten gehen und sollte nur einige Millisekunden bis 100 Millisekunden Verzögerung aufweisen.

Insbesondere bei mobilen, d.h. tragbaren Herz-Lungen-Maschinen ist es von Vorteil, wenn eine derartige Schlauchklemme elektrisch betätigt werden kann, beispielsweise mittels eines Elektromagneten oder eines Elektromotors. Im Prinzip könnte dies dadurch geschehen, dass ein Hubmagnet einen Klemmkörper direkt auf den Schlauch und gegen eine feststehende Haltefläche drückt. Um hierbei die nötige Klemmkraft und Schließgeschwindigkeit für einen zuverlässigen Betrieb erreichen zu können, ist jedoch ein relativ groß dimensionierter Elektromagnet nötig, was gerade bei tragbaren Geräten mit erheblichen Schwierigkeiten hinsichtlich Bauraum, Gewicht und Kosten verbunden ist.

Es ist daher eine Aufgabe der Erfindung, eine elektrisch betätigbare Klemme bereitzustellen, welche mit geringem Aufwand ein zuverlässiges und ausreichend schnelles Abklemmen von Schläuchen ermöglicht.

Diese Aufgabe wird mit einer Klemme mit den Merkmalen des Anspruchs 1 gelöst. Eine erfindungsgemäße Klemme umfasst einen elektrischen Antrieb, zwei Scherenhebel, die gegeneinander um eine Schwenkachse zwischen einer Öffnungsstellung und einer Klemmstellung verschwenkbar sind, und die jeweilige Klemmflächen aufweisen, und eine Umsetzeinrichtung, mittels welcher eine Bewegung des elektrischen Antriebs in eine Bewegung der Scherenhebel umsetzbar ist.

Bei dem elektrischen Antrieb kann es sich beispielsweise um einen einfachen Elektromagneten, einen elektrisch angesteuerten Linearantrieb oder um einen Elektromotor handeln. Die Scherenhebel mit den entsprechenden Klemmflächen ermöglichen ein sicheres und zuverlässiges Abklemmen eines zwischen den Klemmflächen befindlichen Schlauches, wobei die Klemmkraft und die Schließgeschwindigkeit durch entsprechende Wahl der Hebellängen leicht angepasst werden kann. Da der elektrische Antrieb nicht direkt auf die Scherenhebel wirkt, sondern auf die Umsetzeinrichtung, welche ihrerseits eine Bewegung der Scherenhebel herbeiführt, ist die Scherenbewegung von der eigentlichen Antriebsbewegung entkoppelt. Somit ist es insbesondere möglich, mit einem relativ gering dimensionierten elektrischen Antrieb eine hohe Klemmkraft aufzubringen und somit ein zuverlässiges Abklemmen des Schlauches zu bewerkstelligen. Die erfindungsgemäße Klemme kann insbesondere als Schnellschlussklemme ausgebildet sein.

Weitere Ausgestaltungen der Erfindung sind in den untergeordneten Ansprüchen, der Beschreibung sowie der Zeichnung angegeben.

Gemäß einem Ausführungsbeispiel setzt die Umsetzeinrichtung eine Rotationsbewegung des elektrischen Antriebs in eine Schwenkbewegung der Scherenhebel um. Die Umsetzeinrichtung kann also derart ausgestaltet sein, dass die Drehbewegung einer Motorwelle in eine Linearbewegung der Scherenhebel-Enden gewandelt wird. Dadurch wird es möglich, die Klemme mittels eines einfachen und kostengünstigen Elektromotors anzutreiben und auf aufwändige Linearantriebe zu verzichten.

Die Umsetzeinrichtung kann in einem der Klemmstellung der Scherenhebel zugeordneten Zustand selbsthemmend sein. Bei Vorhandensein einer derartigen Selbsthemmung kann nach einem Auslösen der Klemme die Stromversorgung zu dem elektrischen Antrieb unterbrochen werden, ohne befürchten zu müssen, dass sich die Klemme wieder löst und der Kreislauf trotz vorhandener Luftblasen in Gang gesetzt wird. Dadurch dass zum dauerhaften Abklemmen des Schlauches keine fortlaufende Bestromung des elektrischen Antriebs notwendig ist, kann eine beträchtliche Energieeinsparung erzielt werden, was insbesondere bei mobilen Vorrichtungen vorteilhaft ist.

Gemäß einem weiteren Ausführungsbeispiel umfasst die Umsetzeinrichtung einen um eine Rotationsachse drehbaren Rotationskörper, in welchem Kulissen zur Zwangsführung von Endabschnitten der Scherenhebel ausgebildet sind. Eine derartige Kulissenführung ist einfach und kostengünstig in der Herstellung und ermöglicht eine zuverlässige Umsetzung einer Drehbewegung in die gewünschte lineare Scherenhebelbewegung. Die Steuerkurve für den Öffnungs- oder Schließvorgang der Scherenhebel wird dabei durch den Verlauf der Kulissen vorgegeben. Bei den Kulissen kann es sich je nach Ausführungsform um Nuten, Vertiefungen oder Schlitze in dem Rotationskörper handeln. In jeder Kulisse ist der Endabschnitt eines Scherenhebels aufgenommen.

Vorzugsweise ist die Rotationsachse rechtwinklig zu der Schwenkachse angeordnet. Der Rotationskörper dreht sich also in einer Ebene, welche parallel zu der Schwenkachse verläuft. Dadurch wird eine einfache und direkte Umsetzung der Drehbewegung in eine Linearbewegung der Scherenhebel-Enden erzielt.

Die Kulissen können in einer parallel zu der Schwenkachse verlaufenden Ebene einen bogenförmigen Verlauf aufweisen. Insbesondere können zwei einander gegenüber liegende Kulissen vorgesehen sein, welche relativ zueinander versetzt sind. Wesentlich ist, dass sich das zugehörige in der Kulisse aufgenommene Scherenhebel-Ende je nach Laufrichtung von der Rotationsachse entfernt oder sich dieser annähert. Je nach Drehrichtung des Rotationskörpers wird somit eine aufeinander zu gerichtete oder voneinander weg gerichtete Bewegung der Scherenhebel und somit letztendlich ein Betätigen oder Lösen der Klemme herbeigeführt.

Weiterhin können die Kulissen seitliche Führungsflächen mit vorspringenden Halteabschnitten zum Halten von Führungselementen in den Kulissen aufweisen. Bei den Führungselementen kann es sich um Kulissensteine oder ähnliche Komponenten handeln, welche an den jeweiligen Scherenhebeln vorgesehen sind. Die seitlichen Führungsflächen können insbesondere derart geformt sein, dass sie die Kulissensteine teilweise umgreifen und sie so axial fixieren.

Gemäß einer Ausführungsform variieren die Halteabschnitte entlang des Kulissenverlaufs in der Größe. Beispielsweise kann der Halteabschnitt einer innenliegenden Führungsfläche mit zunehmender Entfernung von der Rotationsachse geringer werden, um so die stärkere Schrägstellung des Scherenhebels an dieser Position zu kompensieren.

Gemäß einer weiteren Ausführungsform weisen die Kulissen jeweils an einem der Rotationsachse zugewandten Ende einen Verriegelungsabschnitt auf, welcher ein Festhalten der Scherenhebel in der Klemmstellung bewirkt. Ein derartiger Verriegelungsabschnitt kann beispielsweise in Form eines verstärkt gekrümmten Endbereichs vorgesehen sein. Die verstärkte Krümmung verhindert ein selbsttätiges Zurückgleiten der Scherenhebel in den Kulissen. Es ist vielmehr ein aktives Drehen des Rotationskörpers in Öffnungsrichtung erforderlich, um eine geschlossene Klemme wieder zu lösen. Es könnten auch separat betätigte Riegel oder Rasten vorgesehen sein, um die Scherenhebel bei Erreichen der Klemmstellung in den Kulissen zu verriegeln. Durch die Verriegelungsfunktion kann die Stromversorgung des elektrischen Antriebs unmittelbar nach Erreichen der Klemmstellung unterbrochen werden, um so elektrische Energie zu sparen. Aufgrund der mechanischen Feststellung der Scherenhebel in der Klemmstellung ist also das Aufbringen eines Haltestroms nicht notwendig.

Die Scherenhebel können insbesondere kugelförmige Führungselemente aufweisen, welche in den Kulissen aufgenommen sind. Die Form der Führungselemente oder Kulissensteine ist an die Form der.seitlichen Führungsflächen der zugehörigen Kulisse angepasst. Um die während der Scherenhebelbewegung variierende Entfernung der Führungselemente vom Kreuzungspunkt der Scherenhebel zu berücksichtigen, können die Führungselemente verschiebbar auf Gleitabschnitten der Scherenhebel gelagert sein. Beispielsweise kann jeweils ein Endabschnitt eines Scherenhebels als zylindrischer Stab ausgebildet sein, auf welchem die Kulissensteine entlanggleiten. Die Kulissensteine können zu diesem Zweck mit zylindrischen Durchführungen versehen und auf die Stäbe aufgesteckt sein.

Vorzugsweise sind die Klemmflächen in der Klemmstellung parallel zueinander ausgerichtet. Dies gewährleistet eine gleichmäßige Klemmkraft und somit ein zuverlässiges Abklemmen des Schlauches. Eine entsprechende Ausrichtung der Klemmflächen kann auf einfache Weise durch die Formgebung der Scherenhebel erzielt werden. Bei der Anordnung der Klemmflächen kann ferner berücksichtigt werden, dass der Schlauch auch im abgeklemmten Zustand eine bestimmte Dicke aufweist.

Gemäß einer weiteren Ausführungsform wirkt ein Antriebselement des elektrischen Antriebs auf den Außenumfang eines insbesondere scheibenförmigen Rotationskörpers der Umsetzeinrichtung. Beispielsweise kann die Motorwelle eines Elektromotors über eine entsprechende Antriebsscheibe oder ein Antriebszahnrad eine Drehung des Rotationskörpers herbeiführen. Bei einer derartigen Ausgestaltung findet eine dreifache Kraftübersetzung zwischen dem Antrieb und den Klemmflächen statt. Die erste Kraftübersetzung ergibt sich durch das Angreifen des Antriebs am Außenumfang des Rotationskörpers. Hier kann beispielsweise eine relativ kleine Antriebsscheibe einen relativ großen Rotationskörper drehen, wodurch eine Übersetzung ins Langsame herbeigeführt wird. Die zweite Kraftübersetzung ergibt sich durch die Steuerkurve der Kulissen, deren Verlauf die Kraftumsetzung beeinflusst. Die dritte Kraftübersetzung wird schließlich durch die Scherenhebel selbst herbeigeführt, wobei hier beispielsweise durch entsprechend lange antriebsseitige Scherenhebelabschnitte eine relativ hohe Klemmkraftverstärkung möglich ist. Insgesamt erlaubt die dreifache Kraftübersetzung den Einsatz eines schnellen, aber eher schwachen Elektroantriebs, was hinsichtlich Anschaffungskosten, Bauraum und Gewicht von Vorteil ist.

Gemäß einer Ausgestaltung der Erfindung weist der Rotationskörper eine Außenverzahnung auf, mit welcher das als Verzahnungselement ausgebildete Antriebselement des elektrischen Antriebs kämmt. Die Außenverzahnung kann direkt in den Außenumfang des Rotationskörpers eingeformt sein. Alternativ kann auch beispielsweise ein Zahnrad drehfest mit dem Rotationskörper verbunden sein. In die Außenverzahnung kann beispielsweise ein Ritzel eingreifen, welches direkt an einer Motorwelle angebracht ist. Alternativ kann auch ein Getriebe zwischen die Motorwelle und das Ritzel geschaltet sein. Weiterhin ist es möglich, eine Zahnstange mit der Außenverzahnung in Eingriff zu bringen, welche dann mittels eines Linearantriebs bewegt wird. Eine Kraftübertragung über einen außenverzahnten Rotationskörper arbeitet solide und zuverlässig.

Es kann ein Tragrahmen zur gemeinsamen Lagerung der Umsetzeinrichtung und eines Schwenkzapfens der Scherenhebel vorgesehen sein. Dies vereinfacht die Montage der Klemme in dem zugehörigen medizinischen Gerät. Beispielsweise kann es sich bei dem Tragrahmen um ein U-förmiges Profilelement mit einem Grundplattenabschnitt und zwei abstehenden Trägerabschnitten handeln, wobei der Rotationskörper drehbar in dem Grundplattenabschnitt gelagert ist. Der Schwenkzapfen für die Scherenhebel kann sich dann durch die beiden Trägerabschnitte des Profilelements erstrecken.

Die Erfindung wird nachstehend anhand eines Ausführungsbeispiels unter Bezugnahme auf die anliegende Figur erläutert.

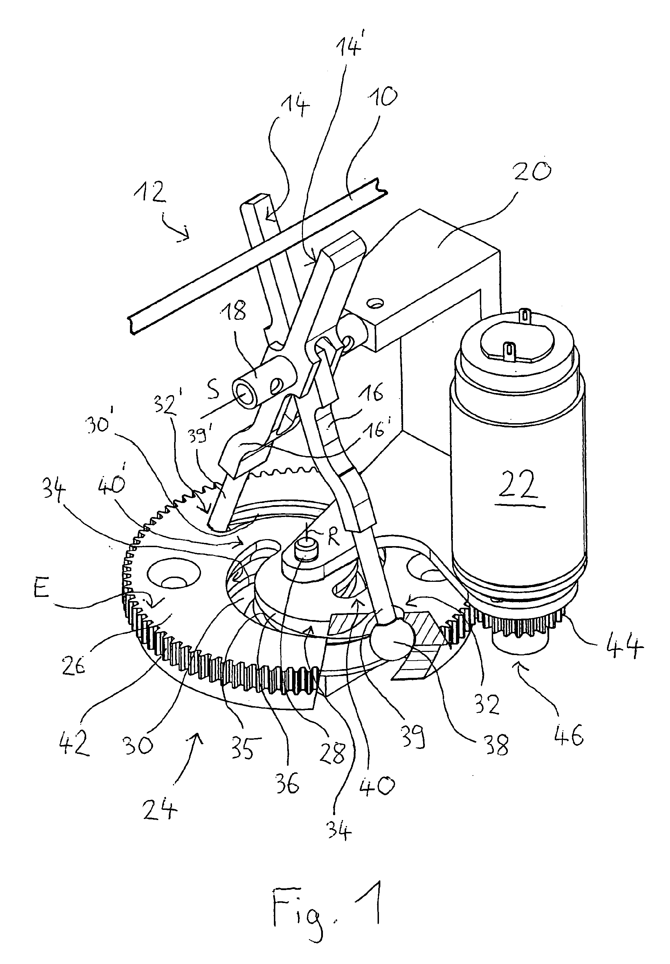

- Fig. 1: zeigt eine teilweise aufgeschnittene Perspektivansicht einer erfindungsgemäßen Klemme.

In der perspektivischen Ansicht von Fig. 1 bezeichnet 10 einen flexiblen Schlauch, der unter bestimmten Umständen abgeklemmt werden soll, beispielsweise einen Bluttransportschlauch innerhalb einer Herz-Lungen-Maschine. Zum bedarfsweisen Abklemmen des Schlauches 10 ist eine Klemme 12 vorgesehen. Sobald von einem nicht gezeigten Detektor erkannt wird, dass in dem durch den Schlauch 10 fließenden Blutstrom eine Luftblase vorhanden ist, muss die Schlauchklemme 12 geschlossen werden, um den Schlauch 10 abzuklemmen. Das Abklemmen wird mittels zweier ebener Klemmflächen 14, 14' bewerkstelligt, welche an jeweiligen Scherenhebeln 16, 16' ausgebildet sind. Die Scherenhebel 16, 16' sind auf einem Schwenkzapfen 18 verschwenkbar gelagert, welcher seinerseits in einem feststehenden Tragrahmen 20 sitzt. Der Tragrahmen 20 ist in der zugehörigen Herz-Lungen-Maschine verbaut, wobei der Schlauch 10 zwischen den Klemmflächen 14, 14' der Scherenhebel 16, 16' hindurchgeführt ist. Der Schwenkzapfen 18 definiert eine Schwenkachse S, um welche die Scherenhebel 16, 16' gegeneinander verschwenkbar sind. Die im Bild dargestellte Stellung der beiden Scherenhebel 16, 16' entspricht einer Öffnungsstellung der Schlauchklemme 12, da hier die Klemmflächen 14, 14' nicht in Kontakt mit dem Schlauch 10 stehen. Bei einer Bewegung der Scherenhebel 16, 16' aufeinander zu drücken die Klemmflächen 14, 14' gegen den Schlauch 10 und bewirken so eine Unterbrechung des Blutflusses durch diesen. Durch ein Aufeinander-zu-Bewegen der beiden Scherenhebel 16, 16' werden diese somit in eine Klemmstellung überführt.

Die Scherenhebel 16, 16' können mittels eines Elektromotors 22 zwischen der Öffnungs- und der Klemmstellung hin- und herbewegt werden, wobei die Umsetzung der Drehbewegung des Elektromotors 22 in die Schwenkbewegung der Scherenhebel 16, 16' durch eine Umsetzeinrichtung 24 bewerkstelligt wird, welche ebenfalls in dem Tragrahmen 20 gelagert ist.

Die Umsetzeinrichtung 24 umfasst einen Rotationskörper 26, welcher mittels eines Drehzapfens 28 drehbar in dem Tragrahmen 20 gelagert ist. Der Drehzapfen 28 definiert eine Rotationsachse R für den Rotationskörper 26, welche rechtwinklig zu der Schwenkachse S angeordnet ist. In dem dargestellten Beispiel weist der Rotationskörper 26 die Form einer Scheibe auf, in welcher zwei gekrümmte Schlitze 30, 30' ausgebildet sind. Die Schlitze 30, 30' verlaufen innerhalb einer Kulissenebene E, welche durch eine Flachseite des scheibenförmigen Rotationskörpers 26 definiert ist und die sich parallel zu der Schwenkachse S erstreckt. Die Scherenhebel 16, 16' sind jeweils an einem Endabschnitt 32, 32' in einem der Schlitze 30, 30' aufgenommen, sodass die Schlitze 30, 30' Kulissen zur Zwangsführung der Scherenhebel 16, 16' bilden. Der bogenförmige Verlauf der Schlitze 30, 30' definiert jeweilige Steuerkurven für eine Bewegung der Scherenhebel 16, 16' aufeinander zu oder voneinander weg.

Die Endabschnitte 32, 32' sind als zylindrische Stäbe 39, 39' ausgebildet, auf welchen kugelförmige Kulissensteine 38 gleitend verschiebbar gelagert sind. Bei einer Drehbewegung des Rotationskörpers 26 führen die Kulissensteine 38 in der jeweiligen Kulisse 30, 30' eine lineare Bewegung in der Kulissenebene E des Rotationskörpers 26 aus. Diese Linearverschiebung der Kulissensteine 38 bewirkt ihrerseits eine Schwenkbewegung der Scherenhebel 16, 16' um die feststehende Schwenkachse S. Die Steuerkurven der Kulissen 30, 30' können derart gewählt sein, dass sich ein bestimmter gewünschter Zusammenhang zwischen der Winkelstellung des Rotationskörpers 26 und dem Öffnungsgrad der Klemme 12 ergibt. Für bestimmte Anwendungen kann es nämlich wünschenswert sein, wenn sich die Öffnungs- oder Schließgeschwindigkeit der Scherenhebel 16, 16' im Verlauf der Bewegung ändert.

Die mit der Schwenkbewegung der Scherenhebel 16, 16' einhergehende Abstandsveränderung zwischen der Schwenkachse S und den Kulissensteinen 38 wird durch eine gleitende Verschiebebewegung der Kulissensteine 38 auf den jeweiligen Stäben 39, 39' kompensiert. Bei einer Drehung des Rotationskörpers 26 ausgehend von der in Fig. 1 dargestellten Öffnungsstellung gegen den Uhrzeigersinn werden somit die Scherenhebel 16, 16' und somit die Klemmflächen 14, 14' aufeinander zu in die Klemmstellung bewegt. Die Neigung und der Endabstand der Klemmflächen 14, 14' sind dabei derart gewählt, dass in der Klemmstellung eine parallele Ausrichtung der Klemmflächen 14, 14' besteht und der Abstand zwischen den Klemmflächen 14, 14' der Restdicke des zusammengedrückten Schlauches 10 entspricht.

Die Schlitze 30, 30' weisen jeweilige seitliche Führungsflächen 34 auf, in welche Nuten 35 mit entsprechenden vorspringenden Halteabschnitten 36 zum Halten der Kulissensteine 38 in den Kulissen 30, 30' eingeformt sind. Die Tiefe der Nuten 35 und somit die Höhe der zugehörigen Halteabschnitte 36 variiert entlang des Kulissenverlaufs derart, dass die Schrägstellung der Stäbe 39, 39' in der jeweiligen Position kompensiert wird. An der im Bild gezeigten, der Öffnungsstellung zugeordneten Position ist an der innenliegenden Führungsfläche 34 nur noch eine relativ schwach ausgeprägte Nut 35 vorhanden.

An einem der Rotationsachse R zugewandten Endbereich der Schlitze 30, 30' ist der Verlauf im Vergleich zu den übrigen Bereichen stärker gekrümmt, so dass sich für jeden Schlitz 30, 30' ein der Klemmstellung zugeordneter Verriegelungsabschnitt 40, 40' ergibt. Die Verriegelungsabschnitte 40, 40' sorgen für ein mechanisches Festhalten der Scherenhebel 16, 16', sobald diese die Klemmstellung erreicht haben. Zum Entriegeln und zum neuerlichen Öffnen der Schlauchklemme 12 ist es somit notwendig, auf den Rotationskörper 26 ein im Uhrzeigersinn gerichtetes Drehmoment auszuüben. Die Umsetzeinrichtung 24 ist also in einem der Klemmstellung der Scherenhebel 16, 16' zugeordneten Zustand selbsthemmend.

Der Antrieb des Rotationskörpers 26 durch den Elektromotor 22 wird nun genauer beschrieben. An dem Außenumfang des Rotationskörpers 26 ist eine Außenverzahnung 42 vorgesehen, welche mit einem direkt an der Motorwelle 46 des Elektromotors 22 angebrachten Ritzel 44 kämmt. Bei einem Bestromen des Elektromotors 22 überträgt dieser ein Drehmoment auf den Rotationskörper 26, wobei sich eine Übersetzung ins Langsame entsprechend den Zähnezahlen der Außenverzahnung 42 sowie des Ritzels 44 ergibt. Die Übersetzung ist an die jeweiligen Anwendungsvorgaben angepasst. Alternativ könnte anstelle des Ritzels 44 auch eine Zahnstange vorgesehen sein, welche von einem Linearantrieb oder Hubmagneten hinund herbewegt wird.

Sobald der vorstehend erwähnte Detektor das Vorhandensein von Luftblasen in dem durch den Schlauch 10 transportierten Blutstrom anzeigt, wird ein entsprechendes Sicherheitssignal erzeugt und es wird darauf beruhend der Elektromotor 22 bestromt. Die Motorwelle 46 dreht das drehfest mit ihr verbundene Ritzel 44, welches seinerseits über die Außenverzahnung 42 den Rotationskörper 26 gegen den Uhrzeigersinn um die Rotationsachse R dreht. Mittels der Schlitze 30, 30' und der in ihnen geführten Kulissensteine 38 wird letztendlich eine aufeinander zu gerichtete Schwenkbewegung der Scherenhebel 16, 16' herbeigeführt, welche ein Abklemmen des Schlauches 10 bewirkt. Da lediglich eine halbe Umdrehung (90 Grad) des Rotationskörpers 26 erforderlich ist, um die Scherenhebel 16, 16' von der Öffnungsstellung in die Klemmstellung zu bewegen, kann ein relativ schnelles Abklemmen erzielt werden. Das Ausgangsdrehmoment des Elektromotors 22 kann relativ gering sein, da über die mehrfache Übersetzung eine hohe Endkraft an den Klemmflächen 14, 14' erreicht werden kann. Sobald die Klemmstellung erreicht ist, kann das Bestromen des Elektromotors 22 gestoppt oder minimiert werden, da die Scherenhebel 16, 16' mittels der Verriegelungsabschnitte 40, 40' festgehalten sind und ein Wiederöffnen der Scherenhebel 16, 16' somit nicht zu befürchten ist. Auf diese Weise können beträchtliche Mengen an elektrischer Energie gespart werden, was insbesondere bei tragbaren - mithin Akku-betriebenen - Herz-Lungen-Maschinen von Vorteil ist.

### Bezugszeichenliste

- 10: Schlauch
- 12: Schlauchklemme
- 14, 14': Klemmfläche
- 16, 16': Scherenhebel
- 18: Schwenkzapfen
- 20: Tragrahmen
- 22: Elektromotor
- 24: Umsetzeinrichtung
- 26: Rotationskörper
- 28: Drehzapfen
- 30, 30': Schlitz
- 32, 32': Endabschnitt
- 34: seitliche Führungsfläche
- 35: Nut
- 36: Halteabschnitt
- 38: Kulissenstein
- 39, 39': Stab
- 40, 40': Verriegelungsabschnitt
- 42: Außenverzahnung
- 44: Ritzel
- 46: Motorwelle
- S: Schwenkachse
- R: Rotationsachse
- E: Kulissenebene

## Patentansprüche

1. Elektrisch betätigbare Klemme (12) zum Abklemmen von Schläuchen (10), die umfasst:
einen elektrischen Antrieb (22),
zwei Scherenhebel (16, 16'), die gegeneinander um eine Schwenkachse (S) zwischen einer Öffnungsstellung und einer Klemmstellung verschwenkbar sind, und die jeweilige Klemmflächen (14, 14') aufweisen, und
eine Umsetzeinrichtung (24), mittels welcher eine Bewegung des elektrischen Antriebs (22) in eine Bewegung der Scherenhebel (16, 16') umsetzbar ist.

2. Klemme nach Anspruch 1,
**dadurch gekennzeichnet, dass**
die Umsetzeinrichtung (24) eine Rotationsbewegung des elektrischen Antriebs (22) in eine Schwenkbewegung der Scherenhebel (16, 16') umsetzt.

3. Klemme nach Anspruch 1 oder 2,
**dadurch gekennzeichnet, dass**
die Umsetzeinrichtung (24) in einem der Klemmstellung der Scherenhebel (16, 16') zugeordneten Zustand selbsthemmend ist.

4. Klemme nach zumindest einem der vorstehenden Ansprüche,
**dadurch gekennzeichnet, dass**
die Umsetzeinrichtung (24) einen um eine Rotationsachse (R) drehbaren Rotationskörper (26) umfasst, in welchem Kulissen (30, 30') zur Zwangsführung von Endabschnitten (32, 32') der Scherenhebel (16, 16') ausgebildet sind.

5. Klemme nach Anspruch 4,
**dadurch gekennzeichnet, dass**
die Rotationsachse (R) rechtwinklig zu der Schwenkachse (S) angeordnet ist.

6. Klemme nach Anspruch 4 oder 5,
**dadurch gekennzeichnet, dass**
die Kulissen (30, 30') in einer parallel zu der Schwenkachse (S) verlaufenden Ebene (E) einen bogenförmigen Verlauf aufweisen.

7. Klemme nach einem der Ansprüche 4 bis 6,
**dadurch gekennzeichnet, dass**
die Kulissen (30, 30') seitliche Führungsflächen (34) mit vorspringenden Halteabschnitten (36) zum Halten von Führungselementen (38) in den Kulissen (30, 30') aufweisen.

8. Klemme nach Anspruch 7,
**dadurch gekennzeichnet, dass**
die Halteabschnitte (36) entlang des Kulissenverlaufs in der Größe variieren.

9. Klemme nach einem der Ansprüche 4 bis 8,
**dadurch gekennzeichnet, dass**
die Kulissen (30, 30') jeweils an einem der Rotationsachse (R) zugewandten Ende einen Verriegelungsabschnitt (40, 40') aufweisen, welcher ein Festhalten der Scherenhebel (16, 16') in der Klemmstellung bewirkt.

10. Klemme nach einem der Ansprüche 4 bis 9,
**dadurch gekennzeichnet, dass**
die Scherenhebel (16, 16') insbesondere kugelförmige Führungselemente (38) aufweisen, welche in den Kulissen (30, 30') aufgenommen sind.

11. Klemme nach zumindest einem der vorstehenden Ansprüche, **dadurch gekennzeichnet, dass**
Führungselemente (38) verschiebbar auf Gleitabschnitten (39, 39') der Scherenhebel (16, 16') gelagert sind.

12. Klemme nach zumindest einem der vorstehenden Ansprüche,
**dadurch gekennzeichnet, dass**
die Klemmflächen (14, 14') in der Klemmstellung parallel zueinander ausgerichtet sind.

13. Klemme nach zumindest einem der vorstehenden Ansprüche,
**dadurch gekennzeichnet, dass**
ein Antriebselement (44) des elektrischen Antriebs (22) auf den Außenumfang eines insbesondere scheibenförmigen Rotationskörpers (26) der Umsetzeinrichtung (24) einwirkt.

14. Klemme nach Anspruch 13,
**dadurch gekennzeichnet, dass**
der Rotationskörper (13) eine Außenverzahnung (42) aufweist, mit welcher das als Verzahnungselement ausgebildete Antriebselement (44) des elektrischen Antriebs (22) kämmt.

15. Klemme nach zumindest einem der vorstehenden Ansprüche, **dadurch gekennzeichnet, dass**
ein Tragrahmen (20) zur gemeinsamen Lagerung der Umsetzeinrichtung (24) und eines Schwenkzapfens (18) der Scherenhebel (16, 16') vorgesehen ist.
